(19) Europäisches Patentamt
European Patent Office
Office européen des brevets

(11) EP 1 322 593 B1

(12) # EUROPÄISCHE PATENTSCHRIFT

(45) Veröffentlichungstag und Bekanntmachung des Hinweises auf die Patenterteilung:
**28.07.2004 Patentblatt 2004/31**

(21) Anmeldenummer: **01985699.6**

(22) Anmeldetag: **28.09.2001**

(51) Int Cl.[7]: **C07C 217/74**, C07D 333/32

(86) Internationale Anmeldenummer:
**PCT/EP2001/011276**

(87) Internationale Veröffentlichungsnummer:
**WO 2002/026694 (04.04.2002 Gazette 2002/14)**

(54) **O-SUBSTITUIERTE 6-METHYL-TRAMADOL-DERIVATE**
O-SUBSTITUTED 6-METHYL-TRAMADOL DERIVATIVES
DERIVES DE 6-METHYL-TRAMADOL O-SUBSTITUES

(84) Benannte Vertragsstaaten:
**AT BE CH CY DE DK ES FI FR GB GR IE IT LI LU MC NL PT SE TR**
Benannte Erstreckungsstaaten:
**LT LV RO SI**

(30) Priorität: **29.09.2000 DE 10049483**

(43) Veröffentlichungstag der Anmeldung:
**02.07.2003 Patentblatt 2003/27**

(73) Patentinhaber: **Grünenthal GmbH**
**52078 Aachen (DE)**

(72) Erfinder:
- **BUSCHMANN, Helmut**
**08950 Esplugues de Llobregat (ES)**
- **KÖGEL, Babette-Yvonne**
**52379 Langerwehe-Hamich (DE)**
- **FRIDERICHS, Elmar**
**52223 Stolberg (DE)**
- **KAULARTZ, Dagmar**
**52222 Stolberg (DE)**

(56) Entgegenhaltungen:
**EP-A- 0 780 369**
**EP-A- 0 786 450**
**US-A- 4 155 935**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft O-substituierte 6-Methyl-Tramadol-Derivate, Verfahren zu deren Herstellung, Arzneimittel enthaltend diese Verbindungen und die Verwendung von O-substituierten 6-Methyl-Tramadol-Derivaten zur Herstellung von Arzneimitteln zur Behandlung von Schmerz.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

EP-A-0 780 369 offenbart 1-Phenyl-2-dimethylaminomethyl-4-Alkyl-cyclohexanol Verbindungen, die analgetische Wirkungen aufweisen.

Eine der Erfindung zugrundeliegende Aufgabe bestand darin, neue analgetisch wirksame Substanzen zur Verfügung zu stellen, die sich zur Schmerztherapie - insbesondere akuter aber auch chronischer und neuropathischer Schmerzen - eignen.

Gegenstand der Erfindung sind daher O-substituierte 6-Methyl-Tramadol-Derivate der allgemeinen Formel I,

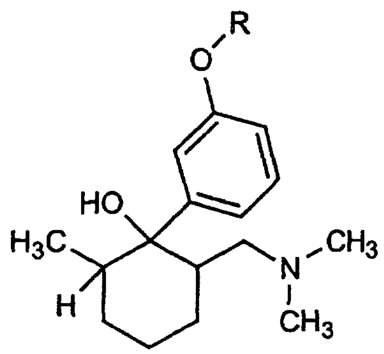

I

, worin

R ausgewählt ist aus

H; $C_{1-3}$-Alkyl, gesättigt oder ungesättigt, verzweigt oder unverzweigt, unsubstituiert oder substituiert; $-CH_3-C_{4-6}$-Cycloalkyl, $C_{4-6}$-Cycloalkyl oder Thiophenyl;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

Die erfindungsgemäßen Substanzen zeigen eine ausgeprägte analgetische Wirkung.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht $C_{1-2}$-Alkyl für C1- oder C2-Alkyl, $C_{1-3}$-Alkyl für C1-, C2- oder $C_3$-Alkyl, $C_{1-4}$-Alkyl für C1-, C2-, C3- oder C4-Alkyl, $C_{1-5}$-Alkyl für C1-, C2-, C3-, C4- oder C5-Alkyl, $C_{1-6}$-Alkyl für C1-, C2-, C3-, C4-, C5- oder C6-Alkyl, $C_{1-7}$-Alkyl für C1-, C2-, C3-, C4-, C5-, C6- oder C7-Alkyl, $C_{1-8}$-Alkyl für C1-, C2-, C3-, C4-, C5-, C6-, C7- oder C8-Alkyl, $C_{1-10}$-Alkyl für C1-, C2-, C3-, C4-, C5-, C6-, C7-, C8,- C9- oder C10-Alkyl und $C_{1-18}$-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht $C_{3-4}$-Cycloalkyl für C3- oder C4-Cycloalkyl, $C_{3-5}$-Cycloalkyl für C3-, C4- oder C5-Cycloalkyl, $C_{3-6}$-Cycloalkyl für C3-, C4-, C5- oder C6-Cycloalkyl, $C_{3-7}$-Cycloalkyl für C3-, C4-, C5-, C6- oder C7-Cycloalkyl, $C_{3-8}$-Cycloalkyl für C3-, C4-, C5-, C6-, C7- oder C8-Cycloalkyl, $C_{4-5}$-Cycloalkyl für C4- oder C5-Cycloalkyl, $C_{4-6}$-Cycloalkyl für C4-, C5- oder

C6-Cycloalkyl, $C_{4-7}$-Cycloalkyl für C4-, C5-, C6- oder C7-Cycloalkyl, $C_{5-6}$-Cycloalkyl für C5- oder C6-Cycloalkyl und $C_{5-7}$-Cycloalkyl für C5-, C6- oder C7-Cycloalkyl. Unter den Begriff Cycloalkyl fallen auch ein- oder mehrfach, vorzugsweise einfach, ungesättigte Cycloalkyle, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch $CHF_2$, $CF_3$, $CH_2OCH_3$ oder $CH_2OH$.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl, Br, I, $NH_2$, SH, $OCH_3$ oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von $CF_3$ oder an verschiedenen Stellen wie im Falle von $-CH(OH)-CH=CH-CHCl_2$. Besonders bevorzugte Substituenten sind hier F, Cl, $OCH_3$ und OH. In Bezug auf Cycloalkyl kann der Wasserstoffrest auch durch $OC_{1-3}$-Alkyl oder $C_{1-3}$-Alkyl (jeweils ein- oder mehrfach substituiert oder unsubstituiert), insbesondere Methyl, Ethyl, n-Propyl, i-Propyl, $CF_3$ oder Ethoxy, ersetzt sein.

Unter dem Begriff $(CH_2)_{3-6}$ ist $-CH_2-CH_2CH_2-$, $-CH_2-CH_2-CH_2-CH_2-$, $-CH_2CH_2-CH_2-CH_2-CH_2-$ und $CH_2-CH_2-CH_2-CH_2-CH_2-CH_2-$ zu verstehen, unter $(CH_2)_{1-4}$ ist $-CH_2-$, $-CH_2-CH_2-$, $-CH_2-CH_2-CH_2-$ und $-CH_2-CH_2-CH_2-CH_2-$ zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5 ] thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert - wenn nicht ausdrücklich anders definiert - die Substitution des Aryls oder Heteroaryls mit OH, F, Cl Br, I, $NH_2$, SH, $CF_3$, $CH_2F$, $CHF_2$, CN, $NO_2$, $C_{1-6}$-Alkyl (gesättigt), $C_{1-6}$-Alkoxy oder $C_{2-6}$-Alkylen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und ionen zu verstehen, insbesondere Komplexe, die über ionische Wechselwirkungen komplexiert sind. insbesondere versteht man darunter physiologisch verträgliche Salze mit Kationen oder Basen und physiologisch verträgliche Salze mit Anionen oder Säuren.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit $NH_4^+$, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure. Besonders bevorzugt ist das Hydrochlorid-Salz.

In einer bevorzugten Ausführungsform der Erfindung ist bei den erfindungsgemäßen O-substituierten 6-Methyl-Tramadol-Derivaten gemäß Formel I

R ausgewählt aus

H; $C_{1-3}$-Alkyl, gesättigt oder ungesättigt, unverzweigt, unsubstituiert oder einfach, vorzugsweise mit $OCH_3$, substituiert; $-CH_3-C_{4-6}$-Cycloalkyl oder $C_{4-6}$-Cycloalkyl, gesättigt und unsubstituiert; Thiophenyl, unsubstituiert;

vorzugsweise R ausgewählt aus

H, $-CH_3$, $-C_2H_5$, $-CH_2-CH=CH_2$, $-CH_2-CH_2-O-CH_3$, $-C{\equiv}CH$; Cyclobutyl, Cyclopentyl, $-CH_3$-Cyclobutyl oder Thiophenyl, jeweils unsubstituiert;

insbesondere R ausgewählt aus

H, $-CH_3$, $-C_2H_5$, $-CH_2-CH=CH_2$, $-C{\equiv}CH$; Cyclobutyl, Cyclopentyl, oder $-CH_3$-Cyclobutyl, jeweils unsubstituiert.

In einer weiteren bevorzugten Ausführungsform der Erfindung ist bei den erfindungsgemäßen O-substituierten 6-Methyl-Tramadol-Derivaten gemäß Formel I R ausgewählt aus Wasserstoff.

In einer bevorzugten Ausführungsform der Erfindung sind die erfindungsgemäßen O-substituierten 6-Methyl-Tramadol-Derivate ausgewählt aus der folgenden Gruppe:

- 2-Dimethylaminomethyl-1-(3-methoxy-phenyl)-6-methyl-cyclohexanol
- 3-(2-Dimethylaminomethyl-1-hydroxy-6-methyl-cyclohexyl)-phenol
- 2-Dimethylaminomethyl-1-(3-ethoxy-phenyl)-6-methyl-cyclohexanol
- 1-(3-Allyloxy-phenyl)-2-dimethylaminomethyl-6-methyl-cyclohexanol
- 1-(3-Cyclopentyloxy-phenyl)-2-dimethylaminomethyl-6-methylcyclohexanol
- 2-Dimethylaminomethyl-1-[3-(2-methoxy-ethoxy)-phenyl]-6-methylcyclohexanol
- 1-(3-Cyclobutylmethoxy-phenyl)-2-dimethylaminomethyl-6-methylcyclohexanol
- 1-(3-Cyclobutoxy-phenyl)-2-dimethylaminomethyl-6-methyl-cyclohexanol
- 2-Dimethylaminomethyl-1-(3-ethynyloxy-phenyl)-6-methyl-cyclohexanol oder
- 2-Dimethylaminomethyl-6-methyl-1-[3-(thiophen-2-yloxy)-phenyl]-cyclohexanol

vorzugsweise ausgewählt aus

- 2-Dimethylaminomethyl-1-(3-methoxy-phenyl)-6-methyl-cyclohexanol oder
- 3-(2-Dimethylaminomethyl-1-hydroxy-6-methyl-cyclohexyl)-phenol,

insbesondere ausgewählt aus

- 3-(2-Dimethylaminomethyl-1-hydroxy-6-methyl-cyclohexyl)-phenol;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, insbesondere der Hydrochlorid-, Bishydrochlorid- oder Natriumsalze.

In einer bevorzugten Ausführungsform der Erfindung liegen die erfindungsgemäßen O-substituierten 6-Methyl-Tramadol-Derivate in einer Stereoisomerenform nach Formel Ia vor:

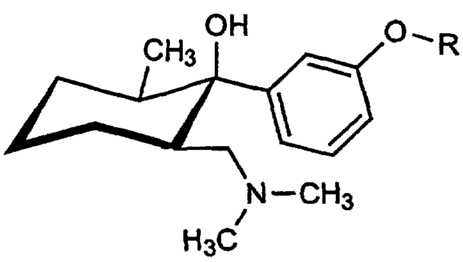

**Ia**

In einer bevorzugten Ausführungsform der Erfindung sind die erfindungsgemäßen O-substituierten 6-Methyl-Tramadol-Derivate ausgewählt aus dem (RS,RS,RS)-Razemat, dem (-)-(S,S,S)- oder (+)-(R,R,R)-Enantiomer oder dem (RS,SR,RS)-Razemat von 3-(2-Dimethylaminomethyl-1-hydroxy-6-methyl-cyclohexyl)-phenol, vorzugsweise

dem dem (-)-(S,S,S)- oder (+)-(R,R,R)-Enantiomer von 3-(2-Dimethylaminomethyl-1-hydroxy-6-methyl-cyclohexyl)-phenol, insbesondere dem (-)-(S,S,S)-Enantiomer, oder ausgewählt sind aus (-)-(1 S,2S,6S)-3-(2-Dimethylaminomethyl-1-hydroxy-6-methyl-cyclohexyl)-phenol,

vorzugsweise in Form der freien Base; oder in Form der Salze, vorzugsweise der physiologisch verträglichen Salze, insbesondere des Hydrochloridsalzes; oder in Form der Solvate, insbesondere der Hydrate.

Die erfindungsgemäßen Substanzen sind toxikologisch unbedenklich, so daß sie sich als pharmazeutischer Wirkstoff in Arzneimitteln eignen. Ein weiterer Gegenstand der Erfindung sind daher Arzneimittel enthaltend wenigstens ein erfindungsgemäßes O-substituiertes 6-Methyl-Tramadol-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen O-substituierten 6-Methyl-Tramadol-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße O-substituierte 6-Methyl-Tramadol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen O-substituierten 6-Methyl-Tramadol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 1000 mg/kg, bevorzugt 0,05 bis 5 mg/kg wenigstens eines erfindungsgemäßen O-substituierten 6-Methyl-Tramadol-Derivats appliziert.

Ein weiterer Gegenstand der Erfindung ist die Verwendung eines erfindungsgemäßen O-substituierten 6-Methyl-Tramadol-Derivats zur Herstellung eines Arzneimittels zur Behandlung von Schmerz, insbesondere von neuropathischem, chronischem oder akutem Schmerz; oder zur Behandlung von Migräne, Hyperalgesie und Allodynie, insbesondere thermischer Hyperalgesie, mechanischer Hyperalgesie und Allodynie und Kälte-Allodynie; oder von inflammatorischem oder postoperativem Schmerz.

Ein weiterer Gegenstand der Erfindung ist ein Verfahren zur Behandlung eines nichthumanen Säugetieres oder Menschen, das oder der eine Behandlung medizinisch relevanter Symptome benötigt, durch Verabreichung einer therapeutisch wiksamen Dosis eines erfindungsgemäßen O-substituierten 6-Methyl-Tramadol-Derivats, oder eines erfindungsgemäßen Arzneimittels. Die Erfindung betrifft insbesondere entsprechende Verfahren zur Behandlung von Schmerz, insbesondere von neuropathischem, chronischem oder akutem Schmerz; Migräne, Hyperalgesie und Allodynie, insbesondere thermischer Hyperalgesie, mechanischer Hyperalgesie und Allodynie und Kälte-Allodynie, oder von inflammatorischem oder postoperativem Schmerz.

Ein weiterer Gegenstand der Erfindung ist ein Verfahren zur Herstellung eines erfindungsgemäßen O-substituierten 6-Methyl-Tramadol-Derivats wie in der folgenden Beschreibung und Beispielen ausgeführt. Daher ist ein weiterer Gegenstand der Erfindung ein Verfahren zur Herstellung eines erfindungsgemäßen O-substituierten 6-Methyl-Tramadol-Derivats, bei dem 2-Dimethylaminomethyl-6-methyl-cyclohexanon gemäß Formel II mit einer metallorganischen Verbindung der Formel III

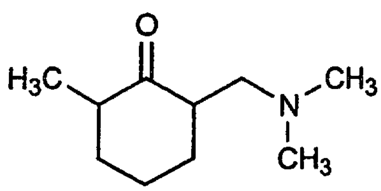

II

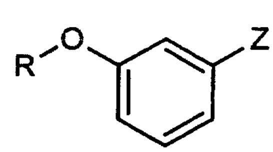

III

in der Z Li bedeutet und R eine der oben für Formel 1 beschriebenen Bedeutungen hat, zu einer Verbindung der

Formel I umgesetzt wird.

**Allgemeine Herstellung der erfindungsgemäßen Verbindungen**

Für die synthetischen Arbeiten sind in der Literatur beschriebene Reaktionen angewandt ( R.C.Larock, Comprehensive Organic Transformations, 2nd edition, Wiley, New York 1999 und dort zitierte Literatur) sowie im Hause bekannte Erfahrungen eingebracht worden.

O-Derivatisierte-6-Methyl-tramadol-Verbindungen der allgemeinen Formel **I** lassen sich durch ein Verfahren herstellen, welches dadurch gekennzeichnet ist, das 2-Dimethylaminomethyl-6-methyl-cyclohexanon **II** mit einer metallorganischen Verbindung der Formel **III**

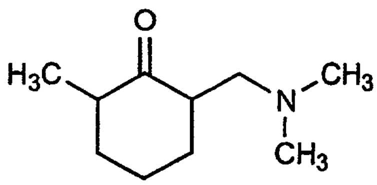

**II**

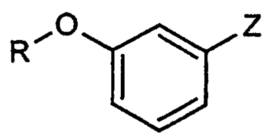

**III**

in der Z für Verbindungen mit R ≠ H MgCl, MgBr, MgI oder Li und für Verbindungen mit R = H Li bedeutet und R eine der oben für Formel **I** beschriebenen Bedeutungen besitzt, zu einer Verbindung der Formel **I** umsetzt.

Alternativ können die erfindungsgemäßen Verbindungen der Formel **I** auch dadurch erhalten werden, das 3-(2-Dimethylaminomethyl-1-hydroxy-6-methyl-cyclohexyl)-phenol (**IV**) mit Halogenverbindungen der Formel **V**, in der X Chlor oder Brom bedeutet, in an sich bekannter Weise mit Basen wie z.B. Kalium-tert-butylat, Natriumhydrid, Kaliumcarbont, Natriumcarbonat, Kaliumhydrogencarbonat, Natriumhydrogencarbonat in Lösungsmitteln wie z.B. Tetrahydrofuran oder Dimethylformamid bei Temperaturen bevorzugt zwischen 0°C und Rückflußtemperatur des Lösungsmittels umgesetzt werden. Die Umsetzung kann auch unter Verwendung von Kaliumhydroxid oder Natriumhydroxid in einem Lösungsmittel wie z.B. Methanol oder Ethanol erfolgen.

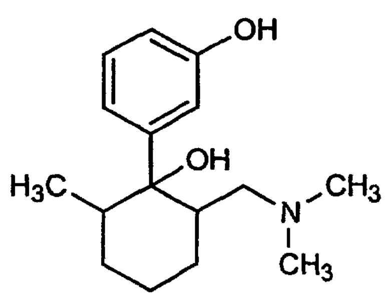

**IV**

R—X

**V**

3-(2-Dimethylaminomethyl-1-hydroxy-6-methyl-cyclohexyl)-phenol der Formel **IV** kann auch dadurch erhalten werden, das 2-Dimethylaminomethyl-1-(3-methoxyphenyl)-6-methyl-cyclohexanol, erhalten durch Umsetzung von 2-Dimethylaminomethyl-6-methyl-cyclohexanon der Formel **II** mit 3-Bromanisol und Magnesium in einer Grignardreaktion, mit selektiven Etherspaltungsreagenzien wie z.B. Diisobutylaluminiumhydrid, Bortrichlorid, Bortribromid oder Methionin in an sich bekannter Weise umsetzt.

Die Umsetzung mit Diisobutylaluminiumhydrid wird bevorzugt in einem aromatischen Kohlenwasserstoff, beispielsweise Toluol, bei einer Temperatur zwischen 60 und 130 °C durchgeführt (Synthesis 1975, 617; DBP 2409990, 2409991 und Chem. Abstr. 84, 59862 (19974)).

Darüber hinaus läßt sich 3-(2-Dimethylaminomethyl-1-hydroxy-6-methyl-cyclohexyl)-phenol der Formel **IV** aus 1-(3-Benzyloxy-phenyl)-2-dimethylaminomethyl-6-methylcyclohexanoldurch durch reduktive Debenzylierung erhalten. Die Debenzylierung führt man in Gegenwart von Platin oder Palladium auf einem Trägermaterial wie Aktivkohle absorbiert als Katalysator in Gegenwart von Wasserstoff in einem Lösungsmittel wie Essigsäure oder einem $C_{1-4}$-

Alkylalkohol bei Drücken von 1 bis 100 bar und Temperaturen von 20 - 100 °C durch.

Die Umsetzung von Dimethylaminomethyl-6-methyl-cyclohexanon 11 mit einer Grignardverbindung der Formel **III**, in der Z MgCl, MgBr oder Mgl bedeutet, oder mit einer lithiumorganischen Verbindung der Formel **III** kann in einem aliphatischen Ether, beispielsweise Diethylether und/oder Tetrahydrofuran, bei Temperaturen zwischen -70 ºC und +60 °C durchgeführt werden. Lithiumorganische Verbindungen der Formel **III**, in der Z Cl, Br oder I bedeutet, lassen sich durch Umsetzung mit beispielsweise einer n-Butyllithium/Hexan-Lösung durch Halogen-Lithiumaustausch erhalten.

Dimethylaminomethyl-6-methyl-cyclohexanon **II** läßt sich nach literaturbekannten Verfahren (Houben-Weyl - Methoden der Organischen Chemie, E21b, 1995, S. 1925-1929; M. Tramontini, L. Angiolini, Mannich Bases, Chemistry and Uses,CRS Press, 1994 und dort zitierte Literatur) herstellen.

Beispielsweise kann Dimethylaminomethyl-6-methyl-cyclohexanon der Formel **II** aus 2-Methylcyclohexanon durch Umsetzung mit Dimethylaminhydrochlorid und Formaldehyd in Eisessig, Wasser oder in einem $C_{1-4}$-Alkylalkohol oder durch Umsetzung mit Dimethylammoniummethylenchlorid in Acetonitril unter Acetylchlorid-Katalyse erhalten werden (Synthesis 1973, 703; Tietze, Eicher, Reaktionen und Synthesen im Organisch Chemischen Praktikum, Thieme Verlag, Stuttgart, 1991, Seite 189).

Die bei der Aminomethylierungsreaktion entstehenden diastereomeren Dimethylaminomethyl-6-methyl-cyclohexanone lassen sich entweder durch säulenchromatographische Trennung oder durch fraktionierte Kristallisation ihrer Hydrochloride aus einem organischem Lösungsmittel wie z.B. 2-Butanon oder Aceton diastereomerenrein erhalten. Möglich ist auch eine Trennung über chirale Säulen und/oder mit chiralen Reagenzien bevorzugt Weinsäure oder substituierter Weinsäure.

**Salzbildung**

Die Verbindungen der Formel I lassen sich mit physiologisch verträglichen Säuren, beispielsweise Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1$\lambda^6$-benzo [d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Acetylsalicylsäure, Hippursäure und/oder Asparaginsäure, in der sich bekannter Weise in ihre Salze überführen. Vorzugsweise wird die Salzbildung in einem Lösungsmittel, beispielsweise Diethylether, Diisopropylether, Essigsäurealkylester, Aceton und/oder 2-Butanon oder auch Wasser durchgeführt. Zur Herstellung der Hydrochloride eignet sich darüber hinaus Trimethylchlorsilan in wäßriger Lösung.

Im folgenden wird die Erfindung weiter durch Beispiele erläutert, ohne sie darauf zu beschränken.

**Beispiele**

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die eingesetzten Chemikalien und Lösungsmittel wurden kommerziell bei den herkömmlichen Anbietem erworben (Acros, Avocado, Aldrich, Fluka, Lancaster, Maybridge, Merck, Sigma, TCI etc.) oder synthetisiert.

Die Analytik erfolgte über ESI-Massenspektrometrie und/oder HPLC und/oder NMR-Spektroskopie.

Die folgenden Beispiele wurden nach den oben angegebenen allgemeinen Herstellungsverfahren hergestellt:

**Liste der Beispiele:**

| Bsp Nr. | R = | Steroisomerie | Name (ohne Angabe der Stereoisomerie) |
|---|---|---|---|
| 1 | $CH_3$ | (RS,RS,RS) | 2-Dimethylaminomethyl-1-(3-methoxy-phenyl)-6-methyl-cyclohexanol |
| 2 | $C_2H_5$ | (RS,RS,RS) | 2-Dimethylaminomethyl-1-(3-ethoxy-phenyl)-6-methyl-cyclohexanol |
| 3 | H | (RS,RS,RS) | 3-(2-Dimethylaminomethyl-1-hydroxy-6-methyl-cyclohexyl)-phenol |
| 4 | $-CH_2-CH=CH_2$ (Allyl) | (RS,RS,RS) | 1-(3-Allyloxy-phenyl)-2-dimethylaminomethyl-6-methyl-cyclohexanol |

(fortgesetzt)

| Bsp Nr. | R = | Steroisomerie | Name (ohne Angabe der Stereoisomerie) |
|---|---|---|---|
| **5** | $CH_3$ | (-)-(S,S,S) | 2-Dimethylaminomethyl-1-(3-methoxy-phenyl)-6-methyl-cyclohexanol |
| **6** | $CH_3$ | (+)-(R,R,R) | 2-Dimethylaminomethyl-1-{3-methoxy-phenyl)-6-methyl-cyclohexanol |
| **7** | Cyclopentyl | (RS,RS,RS) | 1-(3-Cyclopentyloxy-phenyl)-2-dimethylaminomethyl-6-methyl-cyclohexanol |
| **8** | H | (RS,SR,RS) | 3-(2-Dimethylaminomethyl-1-hydroxy-6-methyl-cyclohexyl)-phenol |
| **9** | H | (-)-(S,S,S) | 3-(2-Dimethylaminomethyl-1-hydroxy-6-methyl-cyclohexyl)-phenol |
| **10** | H | (+)-(R,R,R) | 3-(2-Dimethylaminomethyl-1 -hydroxy-6-methyl-cyclohexyl)-phenol |
| **11** | $C_2H_5$ | (-)-(S,S,S) | 2-Dimethylaminomethyl-1-(3-ethoxy-phenyl)-6-methyl-cyclohexanol |
| **12** | $C_2H_5$ | (+)-(R,R,R) | 2-Dimethylaminomethyl-1-(3-ethoxy-phenyl)-6-methyl-cyclohexanol |
| **13** | Cyclopentyl | (-)-(S,S,S) | 1-(3-Cyclopentyloxy-phenyl)-2-dimethylaminomethyl-6-methyl-cyclohexanol |
| **14** | Cyclopentyl | (+)-(R,R,R) | 1-(3-Cyclopentyloxy-phenyl)-2-dimethylaminomethyl-6-methyl-cyclohexanol |
| **15** | $CH_2CH_2$-O-$CH_3$ | (+)-(R,R,R) | 2-Dimethylaminomethyl-1-[3-(2-methoxy-ethoxy)-phenyl]-6-methyl-cyclohexanol |
| **16** | Methylencyclobutyl | (+)-(R,R,R) | 1-(3-Cyclobutylmethoxy-phenyl)-2-dimethylaminomethyl-6-methyl-cyclohexanol |
| **17** | Methylencyclobutyl | (-)-(S,S,S) | 1-(3-Cyclobutylmethoxy-phenyl)-2-dimethylaminomethyl-6-methyl-cyclohexanol |
| **18** | $CH_2CH_2$-O-$CH_3$ | (-)-(S,S,S) | 2-Dimethylaminomethyl-1-[3-(2-methoxy-ethoxy)-phenyl]-6-methyl-cyclohexanol |
| **19** | -C≡CH (Alkinyl) | (+)-(R,R,R) | 2-Dimethylaminomethyl-1-(3-ethynyloxyphenyl)-6-methyl-cyclohexanol |
| **20** | -C≡CH (Alkinyl) | (-)-(S,S,S) | 2-Dimethylarninomethyl-1-(3-ethynyloxyphenyl)-6-methyl-cyclohexanol |
| **21** | Cyclobutyl | (+)-(R,R,R) | 1-(3-Cyclobutoxy-phenyl)-2-dimethylaminomethyl-6-methyl-cyclohexanol |
| **22** | Cyclobutyl | (-)-(S,S,S) | 1-(3-Cyclobutoxy-phenyl)-2-dimethylaminomethyl-6-methyl-cyclohexanol |
| **23** | 2-Thienyl | (RS,RS,RS) | 2-Dimethylaminomethyl-6-methyl-1-[3-(thiophen-2-yloxy)-phenyl]-cyclohexanol |
| **24** | 2-Thienyl | (+)-(R.R,R) | 2-Dimethylaminomethyl-6-methyl-1-[3-(thiophen-2-yloxy)-phenyl]-cyclohexanol |
| **25** | 2-Thienyl | (-)-(S,S,S) | 2-Dimethylaminomethyl-6-methyl-1-[3-(thiophen-2-yloxy)-phenyl]-cyclohexanol |

**Beispiel 26**

**Herstellung von (-)-(1S,2S,6S)-3-(2-Dimethylaminomethyl-1-hydroxy-6-methylcyclohexyl)-phenol, Hydrochlorid**

nach Schema 1

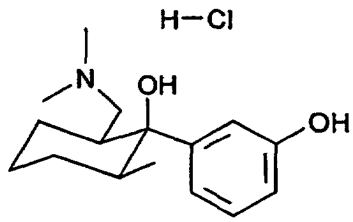

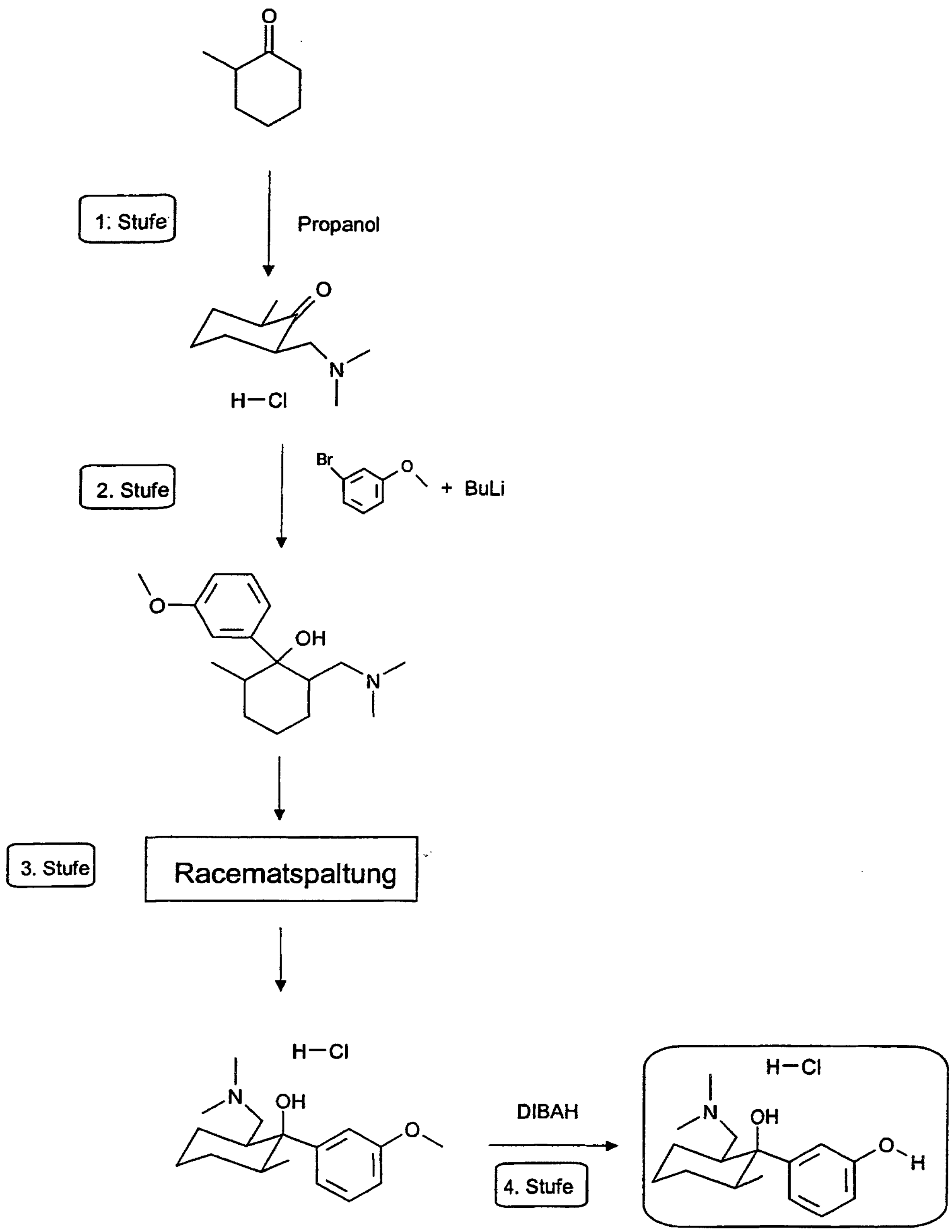
1. Stufe
Propanol
2. Stufe
+ BuLi
3. Stufe
Racematspaltung
DIBAH
4. Stufe

**Beispiel 27**

**Herstellung von (-)-(1S,2S,6S)-3-(2-Dimethylaminomethyl-1-hydroxy-6-methylcyclohexyl)-phenol, Hydrochlorid**

***nach Schema 2***

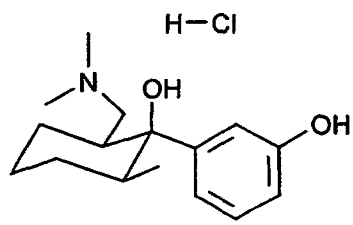

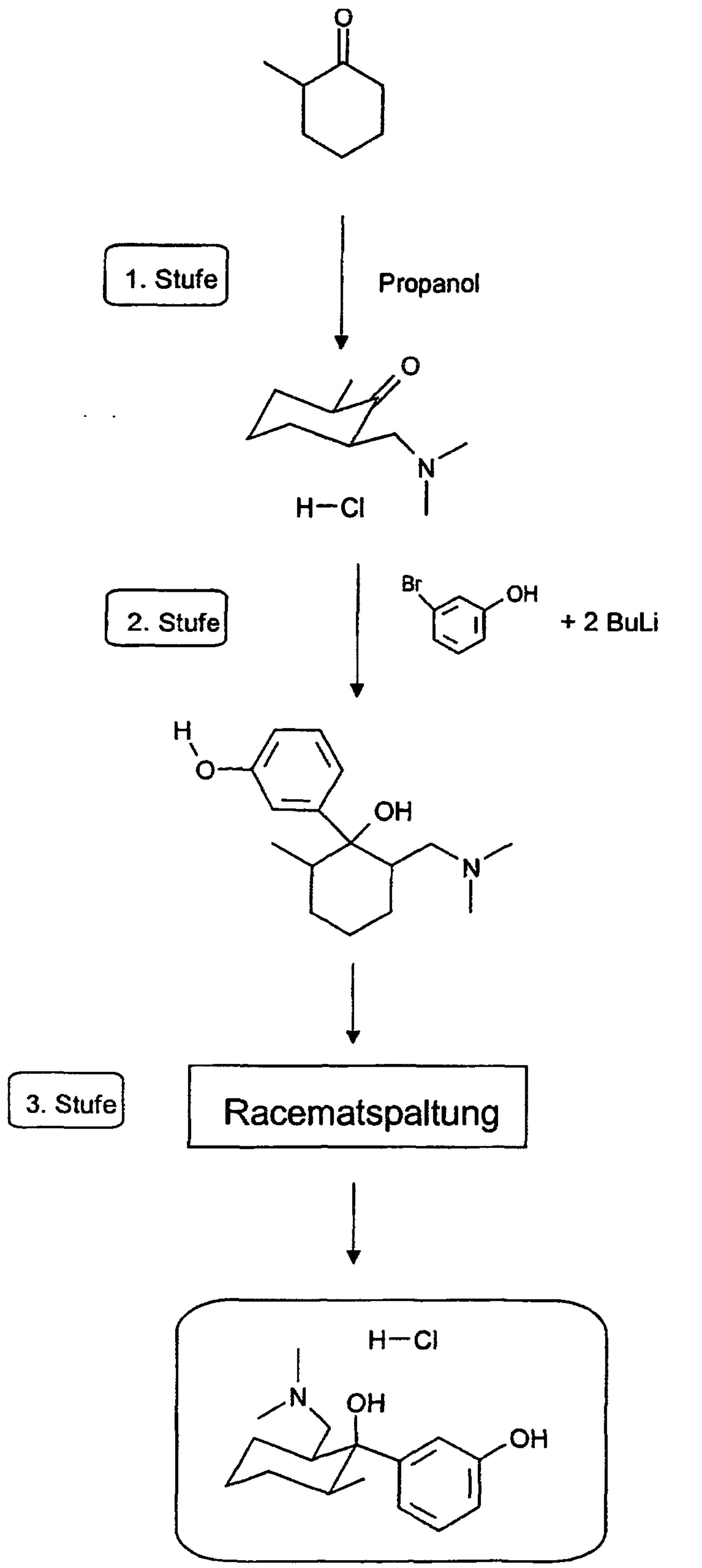
1. Stufe
Propanol
H—Cl
2. Stufe
+ 2 BuLi
3. Stufe
Racematspaltung
H—Cl

**Beispiel 28**

**Herstellung von (-)-(1S,2S,6S)-3-(2-Dimethylaminomethyl-1-hydroxy-6-methylcyclohexyl)-phenol, Hydrochlorid nach folgendem Verfahren:**

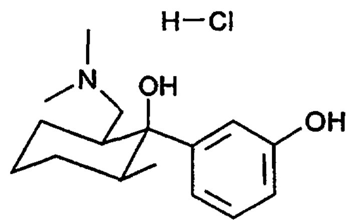

**1. Stufe**

**(2RS,6RS)-2-Dimethylaminomethyl-6-methyl-cyclohexanon, Hydrochlorid**

**Reaktionsgleichung:**

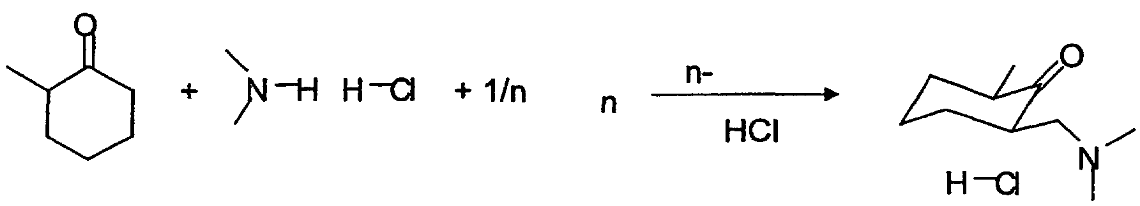


**(112.17)** **(81.55)** **(30.03)**

**Ansatz:** 363 mt = 335,4 g = 3,00 mol 2-Methylcyclohexanon
108 g = 3,60 mol Paraformaldehyd (1,2 Equivalent)
245 g = 3,00 mol Dimethylaminhydrochlorid (1 Equivalent)
1,0 ml konz. $H_2SO_4$
500 ml n-Propanol

**Durchführung:**

2-Methylcyclohexanon, Dimethylaminhydrochlorid und Paraformaldehyd wuden in 500 ml n-Propanol suspendiert und mit 1,0 ml konz. Schwefelsäure versetzt.
Anschließend wurde zwei Stunden unter Rückfluß erhitzt. Nach ca. 30 Minuten war eine klare Lösung entstanden (DC-Reaktionskontrolle; Fließmittel:
Ethylacetat/Methanol = 1 : 1; Probenaufbereitung: 20 µl Reaktionsgemisch + 980 µl Ethanol, je 1 µl aufgetragen). Es ist aber zu beachten, daß beim Erwärmen bei ca 80°C Innentemperatur eine exotherme Reaktion zu beobachten ist.
Das Lösungsmittel wurde am Rotationsverdampfer destillativ entfernt (60 °C Badtemperatur, 100 - 40 Torr).
Der Rückstand wurde in 1500 ml Aceton aufgenommen und mit 75 ml Wasser versetzt. Die Suspension wurde eine Stunde bei 60 °C gerührt und über Nacht bei Raumtemperatur stehen gelassen. Der Rückstand wurde abgesaugt und mit Aceton nachgewaschen (zweimal mit 100 ml). Nach Trocknen im Vakuum konnten 231 g Mannich-Hydrochlorid isoliert werden.

**Ausbeute:** 231 g g (37 % der Theorie)

Als Hauptprodukt ensteht (2RS)-2-Dimethylaminomethyl-2-methyl-cyclohexanon, Hydrochlorid. Die diastereomere 6-Methylverbindung mit axialer Methylgruppe wird nicht gebildet. Weiterhin sind in der Mutterlauge geringe Mengen Bismannich-Kondensationsprodukte enthalten.

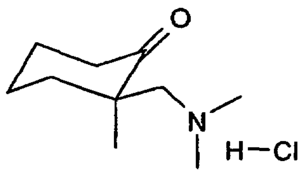
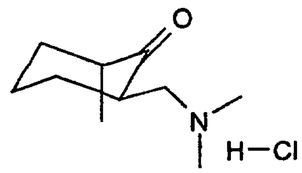
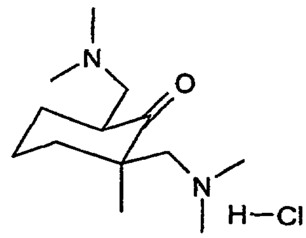

**Charakterisierung:**

| | |
|---|---|
| *Beschreibung:* | weiße kristalline Substanz, frei von sichtbaren Verunreinigungen |
| *Physik. Eigenschaften:* | Schmp.: 164 - 165 °C |
| *Untersuchungsmethoden* | a) GC: AC/GC, Report-Nr. IL 3121-IL 3122<br>CP 9000 Duales System<br>Kanal 0: 25 m Fs. SE 54-CB-1<br>$\nu_{dlt}$ = 250 °C isotherm<br>$\nu_{inj}$ = 230°C<br>$\nu_{ofen}$ = 130°C<br>Carrier: Helium: 100 KPa<br>Range 2<br>Probenaufgabemenge: 1 µl org. Phase;<br>Probenaufbereitung: 20 mg Substanz + 2 Tr. 5 normale NaOH + 200 µl Essigester.<br>b) DC: HPTLC mit Konzentrierungszone (Fa. Merck)<br>Fließmittel: Ethylacetat : Methanol = 1:1;<br>Detektion: Iodkammer, UV-Lampe |
| *Reinheit:* | DC: ein Hauptfleck, >99 %<br>GC: > 98 % |
| *Identität:* | $^{1}$H-NMR, $^{13}$C-NMR entspricht |

**2. Stufe**

**(1RS,2RS,6RS)-3-(2-Dimethylaminomethyl-1-hydroxy-6-methyl-cyclohexyl)-phenol, Hydrochlorid**

**Reaktionsgleichung:**

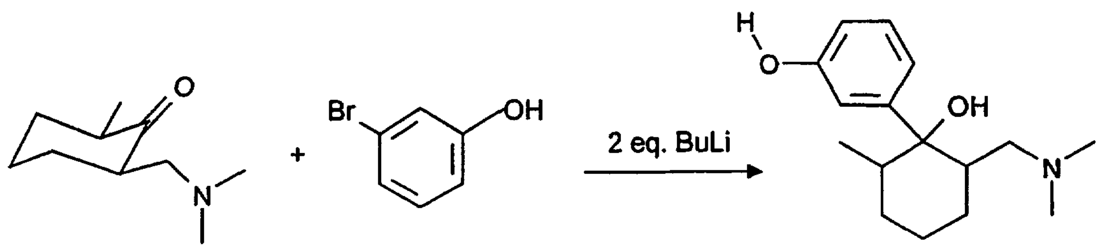


**(169.26/HCl: 205.72)** **(173.02)** **(247.37/HCl: 283.83)**

**Ansatz:** 17,3 g = 100 mmol 3-Bromphenol
125 ml 1,6 molare n-Butyllithium-Lösung in Hexan = 200 mmol
16,9 g =100 mmol (2RS,6RS)-2-Dimethylaminomethyl-6-methylcyclohexanon (Base aus Stufe 1)

**Durchführung:**

17,3 g (=100 mmol) 3-Bromphenol wurden in 80 ml trockenem Tetrahydrofuran gelöst und auf -20 °C gekühlt. Nach Zugabe von 125 ml (200 mmol) 1,6 molarer n-Butyllithiumlösung in Hexan wurde zwei Stunden bei -25 ° gerührt. Anschließend wurden 16,9 g (100 mmol) (2RS,6RS)-2-Dimethylaminomethyl-6-methylcyclohexanon (Base aus Stufe 1), gelöst in 50 ml trockenem Tetrahydrofuran, bei -25 ° zugetropft. Innerhalb von 2,5 Stunden wurde das Reaktionsgemisch auf Raumtemperatur erwärmt.

Zur Aufarbeitung wurden unter Eisbadkühlung 100 ml 5 %ige Salzsäure zugetropft, so daß die Innentemperatur 15 °C nicht überstieg. Nach Phasentrennung wurde die wäßrige Phase dreimal mit 50 ml Ether extrahiert. Die wäßrige Phase wurde mit konzentrierter Natronlauge alkalisiert und erneut mit Ether extrahiert, um das n-Butyl-Additionsprodukt und nicht umgesetzte Mannich-Base abzutrennen. Nach vorsichtiger Neutralisation mit Salzsäure wurde die wäßrige Phase erneut sauer gestellt und anschließend zur Isolierung des Phenols mit Natriumcarbonat alkalisiert und mit Ethylacetat extrahiert. Nach destillativer Entfernung des Lösungsmittels wurde der Rückstand (25 g) in 250 ml Aceton gelöst und mit konz. Salzsäure versetzt. Bei 4 -5°C kristallisierten 12,48 g Hydrochlorid aus.

**Ausbeute:** 12,48 g (44 % der Theorie)

**Charakterisierung:**

| | |
|---|---|
| *Beschreibung:* | weiße kristalline Substanz, frei von sichtbaren Verunreinigungen |
| *Physik. Eigenschaften:* | Schmp.: °C |
| *Untersuchungsmethoden* | DC: HPTLC mit Konzentrierungszone (Fa. Merck)<br>Fließmittel: Ethylacetat : Methanol = 1 : 1;<br>Methylenchlorid : Methanol : Eisessig = 10 : 1 : 1<br>Detektion: Iodkammer, UV-Lampe (254 nm) |
| *Reinheit:* | DC: ein Hauptfleck, >99 % |
| *Identität:* | $^1$H-NMR, $^{13}$C-NMR entspricht |

3. Stufe: Racematspaltung

**(-)-(1 S,2S,6S)-3-(2-Dimethylaminomethyl-1-hydroxy-6-methyl-cyclohexyl)-phenol, Hydrochlorid**

*Racemat*
*(-)-Enantiomer*
*(+) Enantiomer*

**Reaktionsgleichung:**

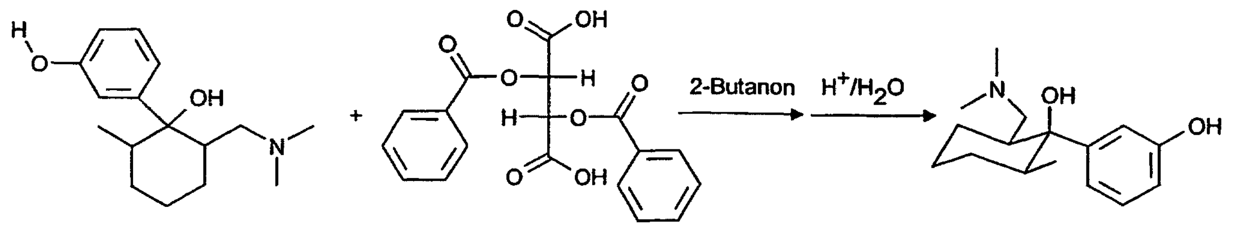


**(247.37/HCl: 283.83)** **(358.31)** **(247.37/HCl: 283.83)**

**Durchführung:**

**a) Fällung mit (+)-Di-O,O'-p-toluyl-weinsäure**

**Ansatz:** 24,7 g = 100 mmol (1RS,2RS,6RS)-3-(2-Dimethylaminornethyl-1-hydroxy-6-methyl-cyclohexyl)-phenol (racemische Base aus Stufe 2)
35,8 g = 100 mmol (+)-Di-O,O'-p-benzoyl-weinsäure

Aus (1RS,2RS,6RS)-3-(2-Dimethylaminomethyl-1-hydroxy-6-methyl-cyclohexyl)-phenol, Hydrochlorid (Stufe 2)) wurde mit Dichlormethan/konzentrierter Natriumcarbonat-Lösung die Base freigesetzt. Nach dem Trocknen der Lösung wurde das Dichlormethan im Vakuum abdestilliert. 24,7 g Racemat wurden in 20 ml 2-Butanon gelöst und unter Rühren mit einer Lösung von 35,8 g (+)-Di-O,O'-p-benzoyl-weinsäure in 400 ml 2-Butanon versetzt. Nach Animpfen begann die Kristallisation des Weinsäuresalzes. Das Reaktionsgemisch wurde über Nacht bei Raumtemperatur stehen gelassen. Der Kristallbrei wurde abgesaugt und mit vorgekühltem 2-Butanon nachgewaschen (2 x 50 ml). Nach Trocknung im Vakuum wurden 25,4 g Weinsäuresalz erhalten. Die destillative Entfernung des Lösungsmittels aus der Mutterlauge ergaben 37 g eines sirupartigen Rückstandes.

**Ausbeute:** 25,4 g Dibenzoylweinsäuresalz
37,0 g Rückstand aus der Mutterlauge

**b) Freisetzen der Basen und Zurückgewinnung der (+)-Di-O, O'-p-benzoylweinsäure**

Das Dibenzoylweinsäuresalz (25 g) wurde in 100 ml Wasser gelöst und mit 5 ml konzentrierter Salzsäure versetzt. Zur Entfernung der (+)-Di-O,O'-p-benzoylweinsäure wurde die wäßrige Phase mit Ether extrahiert (2 x 50 ml). Zur Freisetzung der Base wurde mit 35 ml konzentrierter Natriumhydrogencarbonat-Lösung versetzt und mit Dichlormethan extrahiert (2 x 100 ml). Nach Trocknung der organischen Phase über Natriumsulfat und destillativer Entfernung des Lösungsmittels wurden 9,8 g Base mit einem Enantiomerenüberschuß von >98 % (HPLC) erhalten.

Zur Freisetzung der Base aus der Mutterlauge wurde diese in 150 ml Waaser gelöst und mit 8 ml konzentrierter Salzsäure versetzt. Zur Entfernung der (+)-Di-O,O'-p-benzoyl-weinsäure wurde ebenfalls die wäßrige Phase mit Ether extrahiert (2 x 50 ml) und anschließend mit 57 mi konzentrierter Natriumcarbonat-Lösung alkalisiert. Die Extraktion mit Dichlormethan ergab 14,5 g Base.

Die vereinigten Etherphasen wurden über Natriumsulfat getrocknet. Nach destillativer Entfernung des Lösungsmittels und Trocknung im Vakuum (50° Badtemperatur bei 10-20 Torr) konnten 35 g (+)-Di-O,O'-p-benzoyl-weinsäure zurückgewonnen werden.

**Ausbeute:** 9,8 g Base aus Weinsäuresalz (ee >98 %)
77 g Base aus Mutterlauge (ee= 66 %)
35 (+)-Di-O,O'-p-benzoyl-weinsäure, zurückgewonnen

**Charakterisierung:**

| | |
|---|---|
| *Beschreibung:* | weiße kristalline Substanz, frei von sichtbaren Verunreinigungen |
| *Physik. Eigenschaften:* | Schmp.: 237 - 239 °C<br>$[\alpha]_D^{RT}$ = -36,4 ° (c = 1,01; Methanol) |
| *Untersuchungsmethoden* | a) HPLC:<br>Chiracel OD (mit 250 x 4.6 mm Vorsäule), LKB Pumpe<br>Fließmittel: Hexan : Isopropanol : Diethylamin = 990 : 10 : 1<br>Probenaufgabemenge: 20 μl (0,1%ig im Elutionsmittel)<br>0,75 ml/min<br>UV 273 nm, R.: 0,16<br>b) DC: HPTLC mit Konzentrierungszone (Fa. Merck)<br>Fließmittel: Ethylacetat : Methanol = 1 : 1;<br>Methylenchlorid : Methanol : Eisessig = 10 : 1 : 1<br>Detektion: Iodkammer, UV-Lampe |
| *Reinheit:* | DC: ein Hauptfleck, >99 %<br>HPLC: > 99 % |
| *Optische Reinheit:* | HPLC: ee>99,5<br>(-)-Enantiomer: (+)-Enantiomer 99,75 : 0,25 |
| *Identität:* | $^1$H-NMR, $^{13}$C-NMR, IR, UV entspricht |

**Pharmakologische Untersuchungen**

**Beispiel 29)**

**Writhing-Test an der Maus**

Die analgetische Wirksamkeit der erfindungsgemäßen Verbindungen wurde im Phenylchinon-induzierten Writhing-Test, modifiziert nach I.C. Hendershot, J. Forsaith in J. Pharmacol. Exptl. Ther. 125, 237 (1959), an der Maus untersucht. Dazu wurden männliche NMRI-Mäuse mit einem Gewicht zwischen 25 und 30 g verwendet. Je 10 Tiere erhielten pro Substanzdosis 30 Minuten nach oraler Gabe einer erfindungsgemäßen Verbindung 0,3 ml pro Maus einer 0,02 %igen wäßrigen Phenylchinon-Lösung (Phenylbenzoechinon, Fa. Sigma, Deisenhofen; Herstellung der Lösung unter Zusatz von 5 % Ethanol und Aufbewahrung im Wasserbad bei 45° C) intraperitoneal appliziert. Danach wurden die Tiere einzeln in Beobachtungskäfige gesetzt. Mit Hilfe eines Drucktastenzählers wurde 5-20 Minuten nach Phenylchinongabe die Anzahl der Schmerz-induzierten Streckbewegungen (Writhing-Reaktion = Durchdrücken des Körpers mit Abstrekken der Hinterextremitäten) gezählt. Aus der dosisabhängigen Abnahme der Writhing-Reaktion im Vergleich zu parallel untersuchten Mäusen, denen ausschließlich Phenylchinon appliziert wurde, wurden mittels Regressionsanalyse (Auswertungsprogramm Martens EDV-Service, Eckental) die $ED_{50}$-Werte (effektive Dosis mit 50 %iger Hemmung der Writhing-Reaktion) mit 95 % Vertrauensbereich berechnet. Alle untersuchten erfindungsgemäßen Verbindungen zeigten eine ausgeprägte analgetische Wirkung. Die Ergebnisse sind in der nachfolgenden Tabelle zusammengestellt.

Tabelle:

| Writhing-Hemmung | |
|---|---|
| **Beispiel Nr.** | **$ED_{50}$ [mg/kg per os]** |
| **3** | 2,09 |
| **4** | 9,38 |
| **5** | 11,0 |
| **6** | 6,58 |
| **8** | 14,0 |
| **9** | 19,8 |
| **10** | 5,3 |
| **11** | 21,6 |
| **12** | 4,39 |
| **14** | 26,2 |
| **16** | 32,8 |

**Beispiel 30)**

**Analgesieprüfung im Tail-Flick Test an der Maus**

Die analgetische Wirksamkeit der erfindungsgemäßen Verbindungen wurde im Brennstrahl (Tail-flick) Test an der Maus nach der Methode von D'Amour and Smith (J. Pharm. Exp. Ther. 72, 74 79 (1941) untersucht. Dazu wurden NMRI-Mäuse mit einem Gewicht zwischen 20 - 24 g verwendet. Die Tiere wurden einzeln in spezielle Testkäfige gesetzt und die Schwanzbasis einem focussierten Wärmestrahl einer elektrischen Lampe (Tail-flick Typ 55/12/10.fl, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 3 - 5 Sekunden betrug. Vor Gabe einer erfindungsgemäßen Verbindung wurden die Tiere innerhalb von fünf Minuten zweimal vorgetestet und der Mittelwert dieser Messungen als Vortestmittelwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel:

$$[(T_1 - T_0)/T_2 - T_0)] \times 100$$

Dabei ist die $T_0$ die Latenzzeit vor und $T_1$ die Latenzzeit nach Substanzapplikation, $T_2$ ist die maximale Expositionszeit (12 sec).

Zur Bestimmung der Dosisabhängigkeit wurde die jeweilige erfindungsgemäße Verbindung in 3 - 5 logarithmisch ansteigenden Dosen, die jeweils die Schwellen- und die maximale Wirkdosis einschlossen, appliziert und die $ED_{50}$-Werte mit Hilfe der Regressionsanalyse bestimmt. Die $ED_{50}$-Berechnung erfolgte im Wirkmaximum 20 Minuten nach intravenöser Substanzgabe.

Die untersuchten erfindungsgemäßen Verbindungen zeigten eine ausgeprägte analgetische Wirkung. Die Ergebnisse sind in der nachfolgenden Tabelle zusammengefaßt.

Tabelle:

| Tail-Flick | |
|---|---|
| **Beispiel Nr.** | **$ED_{50}$ [mg/kg i.v.]** |
| **1** | 5,6 |

Tabelle: (fortgesetzt)

| Tail-Flick | |
|---|---|
| **Beispiel Nr.** | **$ED_{50}$ [mg/kg i.v.]** |
| **2** | 11,9 |
| **3** | 2,15 |
| **4** | 20,2 |
| **5** | 42,9 (p.o.) |
| **6** | 7,73 |
| **9** | 14,7 |
| **10** | 0,91 |
| **12** | 13,45 |
| **14** | 20,0 |
| **16** | 21,5 |
| **19** | 14,7 |
| **21** | 30,0 |

## Patentansprüche

**1.** O-substituierte 6-Methyl-Tramadol-Derivate der allgemeinen Formel I,

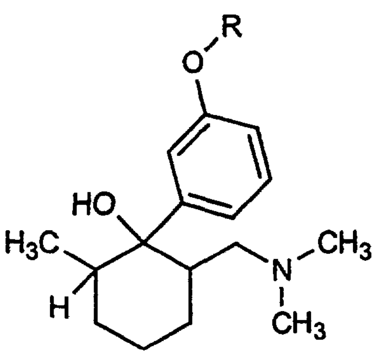

, worin

R ausgewählt ist aus

H; $C_{1-3}$-Alkyl, gesättigt oder ungesättigt, verzweigt oder unverzweigt, unsubstituiert oder substituiert; $-CH_3-C_{4-6}$-Cycloalkyl, $C_{4-6}$-Cycloalkyl oder Thiophenyl;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate.

**2.** O-substituierte 6-Methyl-Tramadol-Derivate gemäß Anspruch 1, **dadurch gekennzeichnet, daß**

R ausgewählt ist aus

H; $C_{1-3}$-Alkyl, gesättigt oder ungesättigt, unverzweigt, unsubstituiert oder einfach, vorzugsweise mit $OCH_3$, substituiert; $-CH_3-C_{4-6}$-Cycloalkyl oder $C_{4-6}$-Cycloalkyl, gesättigt und unsubstituiert; Thiophenyl, unsubstituiert;

vorzugsweise R ausgewählt ist aus
H, $-CH_3$, $-C_2H_5$, $-CH_2CH=CH_2$, $-CH_2-CH_2-O-CH_3$, $-C{\equiv}CH$; Cyclobutyl, Cyclopentyl, $-CH_3$-Cyclobutyl oder Thiophenyl, jeweils unsubstituiert;
insbesondere R ausgewählt ist aus
H, $-CH_3$, $-C_2H_5$, $-CH_2-CH=CH_2$, $-C{\equiv}CH$; Cyclobutyl, Cyclopentyl oder $-CH_3$-Cyclobutyl, jeweils unsubstituiert.

**3.** O-substituierte 6-Methyl-Tramadol-Derivate gemäß einem der Ansprüche 1 oder 2, **dadurch gekennzeichnet, daß** R ausgewählt ist aus H.

**4.** O-substituierte 6-Methyl-Tramadol-Derivate gemäß einem der Ansprüche 1 bis 3, **dadurch gekennzeichnet, daß** das O-substituierte 6-Methyl-Tramadol-Derivat ausgewählt ist aus

- 2-Dimethylaminomethyl-1-(3-methoxy-phenyl)-6-methyl-cyclohexanol
- 3-(2-Dimethylaminomethyl-1-hydroxy-6-rnethyl-cyclohexyl)-phenol
- 2-Dimethylaminomethyl-1-(3-ethoxy-phenyl)-6-methyl-cyclohexanol
- 1-(3-Allyloxy-phenyl)-2-dimethylaminomethyl-6-methyl-cyclohexanol
- 1-(3-Cyclopentyloxy-phenyl)-2-dimethylaminomethyl-6-methylcyclohexanol
- 2-Dimethylaminomethyl-1-[3-(2-methoxy-ethoxy)-phenyl]-6-methylcyclohexanol
- 1-(3-Cyclobutylmethoxy-phenyl)-2-dimethylaminomethyl-6-methylcyclohexanol
- 1-(3-Cyclobutoxy-phenyl)-2-dimethylaminomethyl-6-methyl-cyclohexanol
- 2-Dimethylaminomethyl-1-(3-ethynyloxy-phenyl)-6-methyl-cyclohexanol
- 2-Dimethylaminomethyl-6-methyl-1-[3-(thiophen-2-yloxy)-phenyl]-cyclohexanol

vorzugsweise ausgewählt aus

- 2-Dimethylaminomethyl-1-(3-methoxy-phenyl)-6-methyl-cyclohexanol oder
- 3-(2-Dimethylaminomethyl-1-hydroxy-6-methyl-cyclohexyl)-phenol,

insbesondere ausgewählt aus

- 3-(2-Dimethylaminomethyl-1-hydroxy-6-methyl-cyclohexyl)-phenol;

gegebenenfalls in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren, in einem beliebigen Mischungsverhältnis; in dargestellter Form oder in Form ihrer Säuren oder ihrer Basen oder in Form ihrer Salze, insbesondere der physiologisch verträglichen Salze, oder in Form ihrer Solvate, insbesondere der Hydrate, insbesondere der Hydrochlorid-, Bishydrochlorid- oder Natriumsalze.

**5.** O-substituierte 6-Methyl-Tramadol-Derivate gemäß einem der Ansprüche 1 bis 4, **dadurch gekennzeichnet, daß** die O-substituierten 6-Methyl-Tramadol-Derivate in einer Stereoisomerenform nach Formel Ia vorliegen:

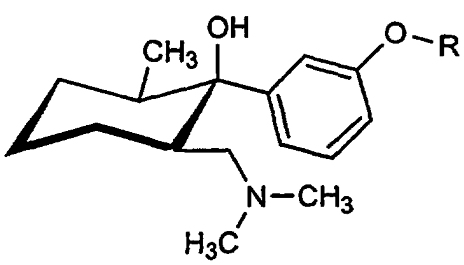

**Ia**

**6.** O-substituierte 6-Methyl-Tramadol-Derivate gemäß einem der Ansprüche 1 bis 5, **dadurch gekennzeichnet, daß**

die O-substituierten 6-Methyl-Tramadol-Derivate ausgewählt sind aus dem (RS,RS,RS)-Razemat, dem (-)-(S,S, S)- oder (+)-(R,R,R)-Enantiomer oder dem (RS,SR,RS)-Razemat von 3-(2-Dimethylaminomethyl-1-hydroxy-6-methyl-cyclohexyl)-phenol, vorzugsweise dem dem (-)-(S,S,S)- oder (+)-(R,R,R)-Enantiomer von 3-(2-Dimethylaminomethyl-1-hydroxy-6-methyl-cyclohexyl)-phenol, insbesondere dem (-)-(S,S,S)-Enantiomer, oder ausgewählt sind aus (-)-(1 S,2S,6S)-3-(2-Dimethylaminomethyl-1 -hydroxy-6-methyl-cyclohexyl)-phenol,
vorzugsweise in Form der freien Base; oder in Form der Salze, vorzugsweise der physiologisch verträglichen Salze, insbesondere des Hydrochloridsalzes; oder in Form der Solvate, insbesondere der Hydrate.

**7.** Arzneimittel enthaltend mindestens ein O-substituiertes 6-Methyl-Tramadol-Derivate gemäß einem der Ansprüche 1 bis 6, sowie gegebenenfalls geeignete Zusatz- und/oder Hilfsstoffe und/oder gegebenenfalls weitere Wirkstoffe.

**8.** Verwendung eines O-substituierten 6-Methyl-Tramadol-Derivats gemäß einem der Ansprüche 1 bis 6 zur Herstellung eines Arzneimittels zur Behandlung von Schmerz, insbesondere von neuropathischem, chronischem oder akutem Schmerz; zur Behandlung von Migräne, Hyperalgesie und Allodynie, insbesondere thermischer Hyperalgesie, mechanischer Hyperalgesie und Allodynie und Kälte-Allodynie; oder von inflammatorischem oder postoperativem Schmerz.

**9.** Verfahren zur Herstellung eines O-substituierten 6-Methyl-Tramadol-Derivats gemäß einem der Ansprüche 1 bis 6, bei dem 2-Dimethylaminomethyl-6-methylcyclohexanon gemäß Formel **II** mit einer metallorganischen Verbindung der Formel **III**

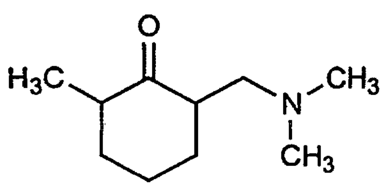

**II**

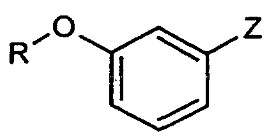

**III**

in der Z Li bedeutet und R eine der oben für Formel **I** beschriebenen Bedeutungen hat, zu einer Verbindung der Formel **I** umgesetzt wird.

## Claims

**1.** O-substituted 6-methyltramadol derivatives of the general formula I,

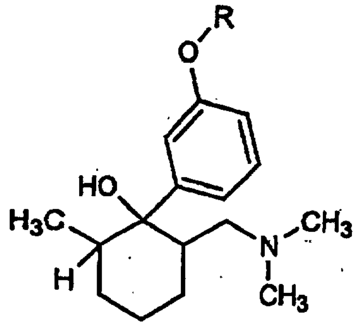

wherein

R is selected from

H; $C_{1-3}$-alkyl that is saturated or unsaturated, branched or unbranched, unsubstituted or substituted; $CH_3$-$C_{4-6}$-cycloalkyl, $C_{4-6}$-cycloalkyl or thiophenyl;

optionally in the form of their racemates, their pure stereoisomers, in particular enantiomers or diastereomers, or

in the form of mixtures of the stereoisomers, in particular of the enantiomers or diastereomers, in an arbitrary mixture ratio; in the prepared form or in the form of their acids or bases or in the form of their salts, in particular of the physiologically compatible salts, or in the form of their solvates, in particular the hydrates.

**2.** O-substituted 6-methyltramadol derivatives according to claim 1, **characterised in that**
R is selected from
H; $C_{1-3}$-alkyl that is saturated or unsaturated, unbranched, unsubstituted or singly substituted, preferably with $OCH_3$; $-CH_3$-$C_{4-6}$-cycloalkyl or $C_{4-6}$-cycloalkyl that is saturated and unsubstituted; thiophenyl that is unsubstituted;
preferably R is selected from
H, $-CH_3$, $-C_2H_5$, $-CH_2-CH=CH_2$, $-CH_2-CH_2-O-CH_3$, $-C\equiv CH$; cyclobutyl, cyclopentyl, $-CH_3$-cyclobutyl or thiophenyl, in each case unsubstituted;
in particular R is selected from
H, $-CH_3$, $-C_2H_5$, $-CH_2-CH=CH_2$, $-C\equiv CH$; cyclobutyl, cyclopentyl or $CH_3$-cyclobutyl, in each case unsubstituted.

**3.** O-substituted 6-methyltramadol derivatives according to one of claims 1 and 2, **characterised in that** R is H.

**4.** O-substituted 6-methyltramadol derivatives according to one of claims 1 to 3, **characterised in that** the O-substituted 6-methyltramadol derivative is selected from

- 2-dimethylaminomethyl-1-(3-methoxyphenyl)-6-methylcyclohexanol
- 3-(2-dimethylaminomethyl-1-hydroxy-6-methylcyclohexyl)-phenol
- 2-dimethylaminomethyl-1-(3-ethoxyphenyl)-6-methylcyclohexanol
- 1-(3-allyloxyphenyl)-2-dimethylaminomethyl-6-methylcyclohexanol
- 1-(3-cyclopentyloxyphenyl)-2-dimethylaminomethyl-6-methylcyclohexanol
- 2-dimethylaminomethyl-1-[3-(2-methoxyethoxy)-phenyl]-6-methylcyclohexanol
- 1-(3-cyclobutylmethoxyphenyl)-2-dimethylaminomethyl-6-methylcyclohexanol
- 1-(3-cyclobutoxyphenyl)-2-dimethylaminomethyl-6-methylcyclohexanol
- 2-dimethylaminomethyl-1-(3-ethynyloxyphenyl)-6-methylcyclohexanol or
- 2-dimethylaminomethyl-6-methyl-1-[3-(thiophen-2-yloxy)-phenyl]-cyclohexanol

and are preferably selected from

- 2-dimethylaminomethyl-1-(3-methoxyphenyl)-6-methylcyclohexanol or
- 3-(2-dimethylaminomethyl-1-hydroxy-6-rnethylcyclohexyl)-phenol

and in particular is

- 3-(2-dimethylaminomethyl-1-hydroxy-6-methylcyclohexyl)-phenol;

optionally in the form of their racemates, their pure stereoisomers, in particular enantiomers or diastereomers, or in the form of mixtures of the stereoisomers, in particular of the enantiomers or diastereomers, in an arbitrary mixture ratio; in the represented form or in the form of their acids or bases or in the form of their salts, in particular of the physiologically compatible salts, or in the form of their solvates, in particular the hydrates, especially the hydrochloride, bishydrochloride or sodium salts.

**5.** O-substituted 6-methyltramadol derivatives according to one of claims 1 to 4, **characterised in that** the O-substituted 6-methyltramadol derivatives are present in a stereoisomeric form according to formula Ia:

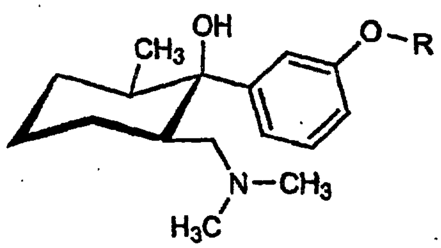

Ia

6. O-substituted 6-methyltramadol derivatives according to one of claims 1 to 5, **characterised in that** the O-substituted 6-methyltramadol derivatives are selected from the (RS,RS,RS) racemate, the (-)-(S,S,S) or (+)-(R,R,R) enantiomer or from the (RS, SR, RS) racemate of 3-(2-dimethylaminomethyl-1-hydroxy-6-methylcyclohexyl)-phenol, preferably the (-)-(S,S,S) or (+)-(R,R,R) enantiomer of 3-(2-dimethylaminomethyl-1-hydroxy-6-methylcyclohexyl)-phenol, in particular the (-)-(S,S,S) enantiomer, or are selected from (-)-(1S, 2S, 6S)-3-(2-dimethylaminomethyl-1-hydroxy-6-methylcyclohexyl)-phenol,
preferably in the form of the free base; or in the form of the salts, preferably the physiologically compatible salts, in particular the hydrochloride salt, or in the form of the solvates, in particular the hydrates.

7. Medicament containing at least one O-substituted 6-methyltramadol derivative according to one of claims 1 to 6, as well as optionally suitable additives and/or auxiliary substances and/or optionally further active constituents.

8. Use of an O-substituted 6-methyltramadol derivative according to one of claims 1 to 6 for the production of a medicament for treating pain, in particular neuropathic, chronic or acute pain; for treating migraine, hyperalgesia and allodynia, in particular thermal hyperalgesia, mechanical hyperalgesia and allodynia and cold-induced allodynia, or inflammatory or post-operative pain.

9. Process for the production of an O-substituted 6-methyltramadol derivative according to one of claims 1 to 6, in which 2-dimethylaminomethyl-6-methylcyclohexanone according to formula **II** is reacted with an organometallic compound of the formula **III**

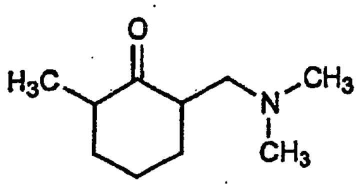

II

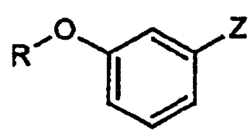

III

in which Z denotes Li and R has one of the meanings described above for formula **I**, to form a compound of the formula **I**.

## Revendications

1. Dérivés de 6-méthyl-tramadol 0-substitués de formule générale I,

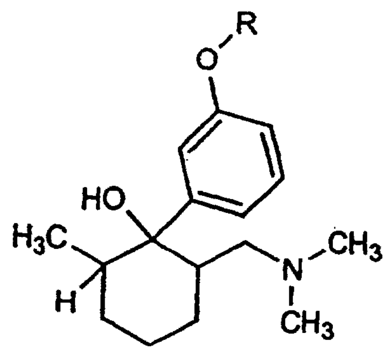

dans laquelle
R est choisi entre
H ; alkyle en $C_1$ à $C_3$, saturé ou non saturé, ramifié ou non ramifié, non substitué ou substitué ; $-CH_3$-(cycloalkyle en $C_4$ à $C_6$), cycloalkyle en $C_4$ à $C_6$ ou thiophényle ;
le cas échéant sous la forme de leurs racémates, de leurs stéréo-isomères purs, en particulier de leurs énantiomères ou de leurs diastéréo-isomères, ou sous forme de mélanges des stéréo-isomères, en particulier des énantiomères ou des diatéréo-isomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates.

**2.** Dérivés de 6-méthyl-tramadol 0-substitués suivant la revendication 1, **caractérisés en ce que** R est choisi parmi
H ; alkyle en $C_1$ à $C_3$, saturé ou non saturé, non ramifié, non substitué ou monosubstitué, avantageusement avec $OCH_3$ ; $-CH_3$-(cycloalkyle en $C_4$ à $C_6$) ou cycloalkyle en $C_4$ à $C_6$, saturé et non substitué ; thiophényle, non substitué ;
R est avantageusement choisi parmi
H, $-CH_3$, $-C_2H_5$, $-CH_2-CH=CH_2$, $-CH_2-CH_2-O-CH_3$, $-C\equiv CH$ ; cyclobutyle, cyclopentyle, $-CH_3$-cyclobutyle ou thiophényle, non substitué dans chaque cas ;
R est en particulier choisi parmi
H, $-CH_3$, $-C_2H_5$, $-CH_2-CH=CH_2$, $-C\equiv CH$ ; cyclobutyle, cyclopentyle ou $-CH_3$-cyclobutyle, non substitué dans chaque cas.

**3.** Dérivés de 6-méthyl-tramadol O-substitués suivant la revendication 1 ou 2, **caractérisés en ce que** H est la valeur choisie pour R.

**4.** Dérivés de 6-méthyl-tramadol O-substitués suivant l'une des revendications 1 à 3, **caractérisés en ce que** le dérivé de 6-méthyl-tramadol O-substitué est choisi entre

- 2-diméthylaminométhyl-1-(3-méthoxyphényl)-6-méthyl-cyclohexanol
- 3-(2-diméthylaminométhyl-1-hydroxy-6-méthyl-cyclohexyl)-phénol
- 2-diméthylaminométhyl-1-(3-éthoxyphényl)-6-méthyl-cyclohexanol
- 1-(3-allyloxyphényl)-2-diméthylaminométhyl-6-méthyl-cyclohexanol
- 1-(3-cyclopentyloxyphényl)-2-diméthylaminométhyl-6-méthyl-cyclohexanol
- 2-diméthylaminométhyl-1-[3-(2-méthoxyéthoxy)-phényl]-6-méthyl-cyclohexanol
- 1-(3-Cyclobutyl-méthoxyphényl)-2-diméthylaminométhyl-6-méthyl-cyclohexanol
- 1-(3-cyclobutoxyphényl)-2-diméthylaminométhyl-6-méthyl-cyclohexanol
- 2-diméthylaminométhyl-1-(3-éthynyloxyphényl)-6-méthyl-cyclohexanol ou
- 2-diméthylaminométhyl-6-méthyl-1-[3-(thiophène-2-yloxy)-phényl]-cyclohexanol

avantageusement choisis entre

- 2-diméthylaminométhyl-1-(3-méthoxyphényl)-6-méthyl-cyclohexanol ou
- 3-(2-diméthylaminométhyl-1-hydroxy-6-méthyl-cyclohexyl)-phénol,

en particulier choisis comme

- 3-(2-diméthylaminométhyl-1-hydroxy-6-méthyl-cyclohexyl)-phénol ;

le cas échéant sous forme de leurs racémates, de leurs stéréo-isomères purs, en particulier de leurs énantiomères ou de leurs diastéréo-isomères, ou sous forme de mélanges des stéréo-isomères, en particulier des énantiomères ou des diastéréo-isomères, dans un rapport de mélange quelconque ; sous la forme représentée ou sous forme de leurs acides ou de leurs bases ou sous forme de leurs sels, en particulier des sels acceptables du point de vue physiologique, ou sous forme de leurs produits de solvatation, en particulier des hydrates, notamment sous forme des chlorhydrates, des bis-chlorhydrates ou des sels de sodium.

**5.** Dérivés de 6-méthyl-tramadol O-substitués suivant l'une des revendications 1 à 4, **caractérisés en ce que** les dérivés de 6-méthyl-tramadol O-substitués se présentent sous une forme stéréo-isomère de formule Ia :

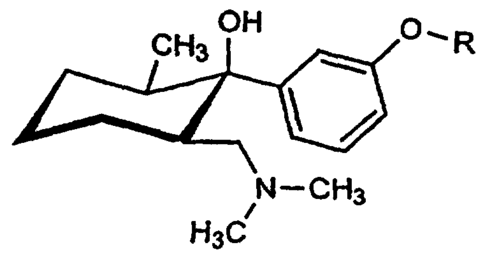

**6.** Dérivés de 6-méthyl-tramadol O-substitués suivant l'une des revendications 1 à 5, **caractérisés en ce que** ces dérivés de 6-méthyl-tramadol O-substitués sont choisis entre le (RS, RS, RS)-racémate, le (-)-(S,S,S)-énantiomère ou le (+)-(R,R,R)-énantiomère ou le (RS,SR,RS)-racémate du 3-(2-diméthyl-aminométhyl-1-hydroxy-6-méthyl-cyclohexyl) -phénol, avantageusement le (-)-(S,S,S)- ou le (+)-(R,R,R)-énantiomère du 3-(2-diméthylaminométhyl-1-hydroxy-6-méthyl-cyclohexyl)-phénol, en particulier le (-)-(S,S,S)-énantiomère, ou bien ils sont choisis comme (-)-(1S,2S,6S)-3-(2-diméthylaminométhyl-1-hydroxy-6-méthyl-cyclohexyl)-phénol,
avantageusement sous forme de la base libre ; ou sous forme des sels, avantageusement des sels acceptables du point de vue physiologique, notamment du chlorhydrate ; ou sous forme des produits de solvatation, en particulier des hydrates.

**7.** Médicament contenant au moins un dérivé de 6-méthyl-tramadol O-substitué suivant l'une des revendications 1 à 6 ainsi que, le cas échéant, des additifs et/ou des substances auxiliaires convenables et/ou éventuellement d'autres substances actives.

**8.** Utilisation d'un dérivé de 6-méthyl-tramadol O-substitué suivant l'une des revendications 1 à 6 pour la préparation d'un médicament destiné au traitement de la douleur, notamment d'une douleur neuropathique, chronique ou aiguë ; au traitement de la migraine, de l'hyperalgésie et de l'allodynie, en particulier de l'hyperalgésie thermique, de l'hyperalgésie et de l'allodynie mécaniques et de l'allodynie due au froid ; ou d'une douleur inflammatoire ou post-opératoire.

**9.** Procédé de préparation d'un dérivé de 6-méthyl-tramadol O-substitué suivant l'une des revendications 1 à 6, dans lequel la 2-diméthylaminométhyl-6-méthyl-cyclohexanone suivant la formule II est amené à réagir avec un composé organométallique de formule III

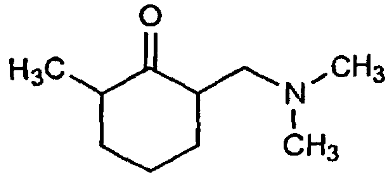

II

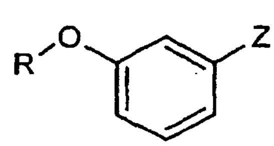

III

où Z désigne le lithium et R a l'une des définitions indiquées ci-dessus pour la formule I, pour former un composé de formule I.